# EUROPEAN PATENT APPLICATION

(11) **EP 2 907 831 A1**
(43) Date of publication of application: **19.08.2015**
(21) Application number: 14155127.5
(22) Date of filing: 14.02.2014
(51) Int. Cl.: C08F 26/06, A61K 8/81, A61K 8/04, C08F 2/14

(54) **Oil-based polymer dispersions for styling applications and cosmetic emulsions**

(71) Applicant: BASF SE, 67056 Ludwigshafen (DE)
(72) Inventor: Hössel, Peter, 67105 Schifferstadt (DE); Böttcher, Axel, 41564 Kaarst (DE); Graham, David, 40213 Düsseldorf (DE); Nguyen-Kim, Son, 69502 Hemsbach (DE); Burakowska-Meise, Ewelina, 68259 Mannheim (DE); Denuell, Wolfgang, 68163 Mannheim (DE); Wendel, Volker, 64342 Seeheim-Jugenheim (DE)
(74) Representative: Reinhardt, Jürgen

(57) **Abstract**

The present invention relates to a process for making a copolymer, wherein the copolymer comprises hydrophilic and/or hydrophobic monomers, comprising copolymerization of the monomers in a one-step process in a cosmetic suitable oil in the presence of common emulsifiers so that a dispersion of the copolymer in the oil is obtained. Furthermore, the present invention relates to the products obtainable by this process.

## Description

The present invention relates to a process for making a copolymer, wherein the copolymer comprises hydrophilic and/or hydrophobic monomers, comprising copolymerization of the monomers in a one-step process in a cosmetic suitable oil in the presence of common emulsifiers so that a dispersion of the copolymer in the oil is obtained. Furthermore, the present invention relates to the products obtainable by this process.

Polymers obtained by precipitation polymerization show good results in thickening of hair styling preparations and cosmetic skin care emulsions (e. g. anionic precipitation polymers based on acrylic acid, vinylpyrrolidone, methyacrylic acid, C16/C18-ethoxylated (25 EO) methacrylates (LUMA).

One significant drawback of this precipitation technology are the high manufacturing costs that result from the process to remove the organic flammable solvents, recycling of solvents and handling of dusty powders in suitable devices including vessel, filter and dryer.

Current inverse emulsion polymerization (resulting in so called liquid dispersion polymers; e. g. Salcare^{®}, Tinovis^{®} and Cosmedia^{®}-grades) leads to thickeners with excellent cosmetic properties. However, the manufacturing process is long and expensive because water needs to be removed by destillation. In addition, the storage stability of these liquid dispersions is limited.

WO 99/29735 discloses a process for making polyvinylpyrrolidone and for making copolymers of vinylpyrrolidone and other monomers. In the process disclosed the monomers are dissolved in oil, e. g. silicon oil or mineral oil. The polymer that is obtained by polymerization of the monomers precipitates from the oil so that a dispersion of the polymer in oil is formed. The examples of WO 99/29735 disclose lauryl methacrylate and acrylic acid as comonomers that are used together with vinylpyrrolidone.

It has now been found that a variety of monomers, both hydrophilic and hydophobic can be copolymerized in a one-step process in the presence of common emulsifiers, which requires no expensive drying or solvent stripping process. The synthesis of those polymers can be carried out in a cosmetic suitable oil.

Key application fields for thickeners based on the described technology are
a) styling products like gels, waxes, pomades, emulsions, lotions, mousses and
b) cosmetic emulsions for skin care and hair care applications.

The use of the described polymers have benefits over conventional market standard polymers. In styling products these benefits are thickening performance, setting properties, humidity resistance either by use of the described polymer alone or in combination with other styling polymers.

In cosmetic skin care emulsions the described benefits are thickening and emulsifying without sacrificing good sensory properties.

## Claims

1. A process for making a copolymer, wherein the copolymer comprises hydrophilic and/or hydrophobic monomers, comprising copolymerization of the monomers in a one-step process in a cosmetic suitable oil in the presence of common emulsifiers so that a dispersion of the copolymer in the oil is obtained.

2. The copolymer obtainable by the process according to claim 1.

3. The dispersion obtainable by the process according to claim 1.

4. The copolymer of claim 2 wherein this copolymer is a thickener.

5. A styling product comprising the copolymer according to claim 2 or the dispersion according to claim 3.

6. The styling product according to claim 5 wherein this styling product is a gel, a wax, a pomade, an emulsion, a lotion or a mousse.

7. A cosmetic emulsion for skin care and/or hair care applications comprising the copolymer according to claim 2 or the dispersion according to claim 3.
